# EUROPÄISCHE PATENTSCHRIFT

(11) **EP 4 479 958 B1**
(45) Veröffentlichungstag und Bekanntmachung des Hinweises auf die Patenterteilung: **12.08.2026**
(21) Anmeldenummer: 23704074.6
(22) Anmeldetag: 03.02.2023
(51) Int. Cl.: G08B 21/06, G01R 33/20

(54) **VERFAHREN UND SYSTEM ZUR ZUSTANDSÜBERWACHUNG EINES BENUTZERS**
METHOD AND SYSTEM FOR MONITORING THE STATE OF A USER
PROCÉDÉ ET SYSTÈME DE SURVEILLANCE DE L'ÉTAT D'UN UTILISATEUR

(30) Priorität: 18.02.2022 DE 102022201710
(43) Veröffentlichungstag der Anmeldung: 25.12.2024
(73) Patentinhaber: Robert Bosch GmbH, 70442 Stuttgart (DE)
(72) Erfinder: STUERNER, Felix Michael, 89077 Ulm (DE); CIPOLLETTI, Riccardo, 73312 Geislingen An Der Steige (DE); WANIEK, Nicolai, 89160 Dornstadt (DE)
(86) Internationale Anmeldenummer: PCT/EP2023/052620
(87) Internationale Veröffentlichungsnummer: WO 2023/156214

(56) Entgegenhaltungen:
- DE-A1- 102011 002 569
- US-A1- 2015 219 732
- US-A1- 2017 305 349

## Beschreibung

Die vorliegende Erfindung betrifft ein Verfahren und ein (helmbasiertes) Sensorsystem zur Zustandsüberwachung eines Benutzers sowie eine Recheneinheit und ein Computerprogramm zu dessen Durchführung.

### Hintergrund der Erfindung

Es sind verschiedene Verfahren und Systeme bekannt, um Fahrerzustände im Auto zu überwachen. So können beispielsweise Augenbewegungen und Blinzelfrequenzen durch Kamerabilder überwacht werden und aus bestimmten Mustern dann auf die Fahrtüchtigkeit des Fahrers geschlossen werden. Dazu muss aber durchgehend ein freies Sichtfeld vorhanden sein, was beispielsweise durch schlechte Lichtverhältnisse, Kopfbewegungen oder Brillen verhindert werden kann. Für Motorradfahrer sind solche Systeme jedoch kaum übertragbar.

Darüber hinaus bieten Messmethoden wie das EEG (Elektroenzephalogramm), bei dem Potentialänderungen über die Kopfhaut verteilt gemessen werden, zuverlässige Hinweise auf den mentalen Zustand einer Person, unter anderem zur Erkennung von Müdigkeit oder Notfällen. Jedoch müssen dabei Elektroden am Kopf korrekt angelegt und für ein gutes Messergebnis mit Kontaktgel versehen werden, so dass ein EEG als Messverfahren im Alltag wie etwa zur Fahrerüberwachung nicht praktikabel ist.

Die DE 10 2011 002 569 A1 offenbart ein Bekleidungsstück, wie bspw. einen Handschuh oder einen Helm für Motorradfahrer, mit einem oder mehreren darin integrierten Sensoren, mit denen die Verfassung des Motorradfahrers überwacht wird. Das Bekleidungsstück umfasst auch einen Signalgeber, mittels dessen dem Fahrer bspw. haptisch, optisch oder akustisch wahrnehmbar signalisiert werden kann, dass seine aktuelle Verfassung schlecht ist und er seine Fahrt besser unterbrechen sollte.

Die US 2017/0305 349 A1 offenbart ein Verfahren, mit dem ein Müdigkeitszustand eines Fahrers erkannt werden kann. Hierzu ist u.a. ein mit Sensoren versehener Helm vorgesehen. Mit Hilfe der Sensoren wird ein EEG zur Erfassung von Konzentrationsschwankungen des Fahrers aufgenommen.

Außerdem sind mittlerweile auch für Motorräder Systeme wie eCall verbreitet, die die Kollisionen und Stürze anhand von geeigneten Sensoren am Motorrad erfassen und bei Bedarf eine automatische Alarmierung von Rettungskräften über das Mobilfunknetz auslösen können. Allerdings kommen diese Systeme erst dann zur Anwendung, wenn ein Unfall bereits eingetreten ist.

### Offenbarung der Erfindung

Erfindungsgemäß werden eine Sensoreinheit und ein Verfahren zur Zustandsüberwachung eines Benutzers sowie eine Recheneinheit und ein Computerprogramm zu dessen Durchführung mit den Merkmalen der unabhängigen Patentansprüche vorgeschlagen. Vorteilhafte Ausgestaltungen sind Gegenstand der Unteransprüche sowie der nachfolgenden Beschreibung.

Gemäß den unabhängigen Ansprüche 1 bzw. 7 wird ein Verfahren bzw. Sensorsystem vorgeschlagen, bei dem Sensordaten im Kopfbereich eines Benutzers während einer Fahrzeugfahrt erfasst werden, die abhängig von einem mentalen oder gesundheitlichen Zustand des Benutzers sind, wobei die Sensordaten über eine oder mehrere Sensoreinheiten erfasst werden, die in einem Helm, der vom Benutzer getragen wird, angeordnet sind. Anschließend wird geprüft, ob die erfassten Sensordaten auf einen unerwünschten oder unbekannten Zustand des Benutzers hinweisen, der die Fahrtüchtigkeit des Benutzers einschränkt. Falls dies der Fall ist, wird eine zustandsabhängige Reaktion ausgelöst.

Darüber hinaus wird in einer Korrektureinheit eine Positionskorrektur für die Sensoreinheiten relativ zum Kopf ermittelt. Dazu werden Fahrtdaten der Fahrzeugfahrt erfasst und eine erwartete Auswirkung der Fahrtdaten auf eine Position des Helms relativ zum Kopf des Benutzers ermittelt. Aus dieser Auswirkung wird dann eine Positionskorrektur für die erfassten Sensordaten bestimmt. Damit können auch leichte Verschiebungen des Helms z.B. durch Beschleunigungskräfte mit berücksichtigt werden. Als Fahrtdaten können zu diesem Zweck beispielsweise eine aktuelle Fahrgeschwindigkeit, eine aktuelle Beschleunigung oder ein vorgegebener Fahrweg auf einer Karte ausgewertet werden.

Die zustandsabhängige Reaktion kann beispielsweise ein Ausgeben eines optischen und/oder akustischen Signals an den Benutzer, ein Übermitteln einer Nachricht über eine Kommunikationsschnittstelle an eine Notrufzentrale und/oder ein Übermitteln einer Nachricht über eine Kommunikationsschnittstelle an andere Fahrzeuge in der Umgebung umfassen. Das Ausgeben eines optischen und/oder akustischen Signals kann durch Signalisierungsmittel erfolgen, die in dem Helm angeordnet sind, z.B. Lautsprecher, ein im Visier integriertes Display oder kleine Warnleuchten.

Wenn hier von "Fahrer", "Fahrzeug", "Fahrt" usw. gesprochen wird, sollen davon alle Arten von Land-, Wasser- und Luftfahrzeugen umfasst sein. Wesentlich ist nur, dass eine Person einen Helm trägt.

Gemäß beispielhaften Ausführungsformen kann das Prüfen der erfassten Sensordaten in einer externen Verarbeitungseinheit außerhalb des Helms als Auswertungseinheit erfolgen, so dass die erfassten Sensordaten an die Auswertungseinheit über ein Kommunikationsmodul in dem Helm übermittelt werden. Dadurch kann eine deutlich größere Rechenleitung vorgehalten werden als in einem Helm.

In allen Varianten kann der unerwünschte Zustand des Benutzers, der durch das System bzw. das Verfahren erkannt werden soll, beispielsweise einer der folgenden sein: Müdigkeit, Einschlafen, erhöhtes Stressniveau, niedrige Konzentration, eine neuronale Erkrankung, einen epileptischen Anfall, einen Schlaganfall.

Als Sensoreinheit kann insbesondere mindestens ein spinresonanz-basiertes Magnetometer zum Erfassen von durch Hirnströme induzierten Hirn-Magnetfeldern verwendet werden. Diese Magnetometer sind in der Lage, hochempfindliche Magnetfeldmessungen bei kompaktem Aufbau durchzuführen.

Als Variante kann ein Gradiometer als Sensoreinheit verwendet werden, das mindestens zwei Magnetometer umfasst, die in einem festen Abstand zueinander angeordnet sind, wobei jedes Magnetometer ein Sensormedium umfasst und dazu eingerichtet ist, eine magnetische Feldstärke an einem Messort im Kopfbereich des Benutzers durch Auslesen einer von der magnetischen Feldstärke abhängigen Spinresonanz in dem Sensormedium zu erfassen, wobei die Sen soreinheit weiter mindestens eine Anregungslichtquelle zum Einstrahlen von Licht in die Sensormedien der Magnetometer umfasst, und mindestens eine Signalverarbeitungseinheit zum Ermitteln eines Magnetfeldgradienten an einer Sensoreinheit als Differenz der Ausgangssignale der zwei Magnetometer der Sensoreinheit und zum Ausgeben der Differenz als Sensordaten der Sensoreinheit. Die Gradiometer-Konfiguration ermöglicht eine Elimination von Hintergrundfeldern und damit eine alltagstaugliche Messung selbst sehr kleiner Magnetfelder.

Eine erfindungsgemäße Recheneinheit ist, insbesondere programmtechnisch, dazu eingerichtet, ein erfindungsgemäßes Verfahren durchzuführen.

Auch die Implementierung eines erfindungsgemäßen Verfahrens in Form eines Computerprogramms oder Computerprogrammprodukts mit Programmcode zur Durchführung aller Verfahrensschritte ist vorteilhaft, da dies besonders geringe Kosten verursacht, insbesondere wenn ein ausführendes Steuergerät noch für weitere Aufgaben genutzt wird und daher ohnehin vorhanden ist. Schließlich ist ein maschinenlesbares Speichermedium vorgesehen mit einem darauf gespeicherten Computerprogramm wie oben beschrieben. Geeignete Speichermedien bzw. Datenträger zur Bereitstellung des Computerprogramms sind insbesondere magnetische, optische und elektrische Speicher, wie z.B. Festplatten, Flash-Speicher, EEPROMs, DVDs u.a.m. Auch ein Download eines Programms über Computernetze (Internet, Intranet usw.) ist möglich. Ein solcher Download kann dabei drahtgebunden bzw. kabelgebunden oder drahtlos (z.B. über ein WLAN-Netz, eine 3G-, 4G-, 5G- oder 6G-Verbindung, etc.) erfolgen.

Weitere Vorteile und Ausgestaltungen der Erfindung ergeben sich aus der Beschreibung und der beiliegenden Zeichnung.

Die Erfindung ist anhand von Ausführungsbeispielen in der Zeichnung schematisch dargestellt und wird im Folgenden unter Bezugnahme auf die Zeichnung beschrieben.

### Kurze Beschreibung der Zeichnungen

Figur 1 zeigt ein Magnetfeld-Gradiometer als Beispiel einer Sensoreinheit, die in einem Sensorsystem eingesetzt werden kann;
Figur 2 zeigt ein beispielhaftes Sensorsystem in einem Helm zur Zustandsüberwachung eines Benutzers; und
Figur 3 zeigt ein Flussdiagramm mit beispielhaften Verfahrensschritten.

### Ausführungsformen der Erfindung

Der mentale oder gesundheitliche Zustand einer Person kann über nicht-invasive Sensoren überwacht werden, die am Kopf des Fahrers bzw. in der Nähe des Kopfes angebracht werden. Für die Zustandsüberwachung von Motorradfahrern oder anderen Helmträgern bietet sich daher insbesondere an, eine oder mehrere Sensoreinheiten in einem Helm anzuordnen. Abhängig von den erfassten Zustandsdaten können verschiedene Reaktionen eingeleitet werden, um die Sicherheit des Fahrers und anderer Verkehrsteilnehmer zu gewährleisten.

Insbesondere können sich aus den Hirnströmen einer Person Hinweise auf verschiedene mentale, körperliche oder gesundheitliche Zustände der Person ergeben. Die elektrische Aktivität in den Hirnneuronen kann aber nicht nur herkömmlich über Elektroden gemessen werden, sondern induziert auch ein schwaches Magnetfeld, das durch hochempfindliche Magnetfeldsensoren erfasst und ausgewertet werden kann. Aus dem zeitlichen Verlauf der induzierten Magnetfelder können dann ebenfalls Rückschlüsse auf die Hirnstrommuster und damit auf verschiedene Zustände einer Person gezogen werden. Die erfassten Magnetfelder können zu diesem Zweck auf der Grundlage bekannter Muster oder Musterparameter oder mit Hilfe trainierter Klassifikatoren ausgewertet werden.

Da die zu messenden Magnetfeldstärken für eine Überwachung der biomagnetischen Hirn-Magnetfelder sehr klein sind, eignen sich als Sensoren für einen derartigen Aufbau insbesondere quantenbasierte Magnetometer, die hochempfindliche Messungen erlauben. Magnetometer dieser Art nutzen optisch gepumpte und/oder optisch detektierte magnetische Resonanzen (optically detected magnetic resonance, ODRM). Dabei wird ausgenutzt, dass unter Einfluss eines äußeren Magnetfelds die Energieniveaus bestimmter Spinzustände ungepaarter Elektronen aufspalten (Zeeman-Effekt). Durch die Aufspaltung der Energieniveaus ergeben sich veränderte Übergänge bei der Relaxation aus angeregten Zuständen, die dann beispielsweise durch optische Anregung und frequenzabhängige Detektion der resultierenden Fluoreszenzstrahlung oder durch Beobachtung optischer Eigenschaften wie der Absorption von Licht gemessen werden können. Aus den gemessenen optischen Parametern kann dann wiederum auf die Magnetfeldstärke geschlossen werden. Beispiele dafür sind unter anderem Dampfzellen-Magnetometer und Diamant-NV-Magnetometer. Es ist jedoch auch denkbar, andere Magnetometer mit vergleichbaren Eigenschaften in die Sensoreinheit zu integrieren.

Diamant-NV-Magnetometer beruhen auf dem optischen Auslesen von Spin-Magnetresonanzen an speziellen Defektzentren in Diamant, insbesondere von Stickstoff-Fehlstellen (NV, nitrogen vacancy), die als Verunreinigungen des Kohlenstoffgitters von Diamant auftreten und auch gezielt eingebracht werden können. Details zu einer möglichen Ausführungsform eines Diamant-NV-Magnetometers finden sich beispielsweise in der DE 10 2018 202 238 A1. Eine aufwändige Kühlung wie etwa bei SQUID-Sensoren ist nicht nötig.

Dampfzellen-Magnetometer beruhen ebenfalls auf der Auslesung von Spinresonanzen. Dabei wird eine Zelle, z.B. eine geschlossene Kavität in einem Silizium-Wafer, mit gasförmigen Atomen, z.B. verdampfte Alkalimetalle wie Kalium, Rubidium oder Cäsium, die ebenfalls ungepaarte Elektronen aufweisen, und gegebenenfalls zusätzlichen Puffergasen gefüllt. Je nach Ausführungsform können auch Mischungen aus Alkali-Dampf und Edelgasen verwendet werden. In anderen Fällen werden reine Edelgase genutzt. Auch bei Dampfzellen-Magnetometern wird eine Spinpolarisierung über optisches Pumpen mit einer Anregungslichtquelle erreicht. Anschließend werden optische Eigenschaften der Atome in der Zelle über die Resonanzeffekte ausgelesen, z.B. mit einer zusätzlichen Auslese-Lichtquelle, anhand derer die Absorption des Ausleselichts durch die Dampfzelle gemessen wird. Dabei können unterschiedliche magnetfeldabhängige Zustandsaufspaltungen beobachtet werden.

Um in einer Alltagsumgebung wie bei der Verwendung eines Helms einsetzbar zu sein, sollen Magnetfelder, die nicht von der Hirnaktivität stammen, aus der Messung möglichst eliminiert werden. Gerade im Bereich von Fahrzeugen entstehen im Hintergrund vergleichsweise hohe und kaum abgeschirmte Magnetfelder, die etwa im Bereich von 10⁻⁶ bis 10⁻⁹ Tesla (Nanotesla) liegen, zusätzlich ist das Erdmagnetfeld im Bereich von 10⁻⁵ Tesla (einige Mikrotesla) vorhanden. Dagegen bewegen sich die biomagnetischen Felder, die hier von Interesse sind, im Bereich von 10⁻¹² Tesla (Picotesla) oder noch darunter.

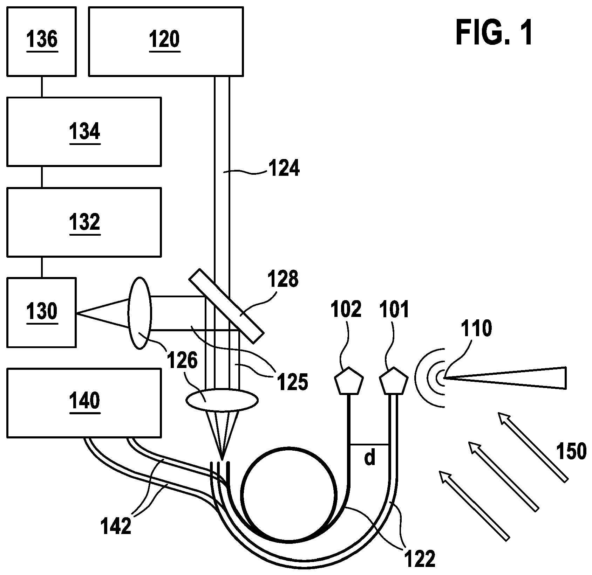

Figur 1 zeigt beispielhaft ein Magnetfeld-Gradiometer, das als Sensoreinheit für ein erfindungsgemäßes System verwendet werden kann. Dazu können mindestens zwei einzelne Magnetometer 101, 102 verwendet werden, die an räumlich unterschiedlichen Stellen angeordnet sind. Aus den erfassten einzelnen Feldsignalen wird eine Differenz gebildet. Durch eine versetzte Anordnung der beiden Sensorköpfe, z.B. der Sensor-Diamanten 101 und 102 eines NV-Zentren-Magnetometers relativ zur Magnetfeldquelle 110, also in diesem Fall zu den verschiedenen Hirnbereichen eines Fahrers, können die an beiden Orten näherungsweise gleich starken Hintergrundfelder bei der Signaldifferenzbildung im Wesentlichen eliminiert werden, während sich die interessierenden schwachen Felder mit dem Quadrat des Abstands von der Quelle verringern. Der Gradient entspricht damit also näherungsweise dem interessierenden schwachen Magnetfeld. Damit entfällt die Notwendigkeit einer aufwendigen magnetischen Abschirmung. Zu diesem Zweck können beispielsweise zwei Magnetometer übereinander in einer axialen Gradiometerkonfiguration, d.h. im Wesentlichen senkrecht zur Schädeloberfläche, angeordnet werden, wobei nur als Beispiel zwischen den beiden Sensorköpfen (z.B. den Sensordiamanten eines NV-Zentren-Magnetometers) ein Abstand d von einigen mm bis cm liegen kann. In anderen Ausführungen können auch zwei Sensorköpfe 101, 102 nebeneinander angeordnet werden. Der Abstand der Sensoreinheit vom Kopf kann ebenfalls im mm- bis cm-Bereich liegen.

Durch Verwendung derselben Lichtquelle 120 für das Anregungslicht 124 und derselben Mikrowellenquelle 140 zur Resonanzerzeugung bei einem NV-Zentren-Magnetometer kann erreicht werden, dass Rauschanteile aus diesen Quellen durch die Differenzsignalbildung automatisch eliminiert werden, da dieses Rauschen bzw. Schwankungen an beiden Sensorköpfen gleich auftritt (common noise rejection). Das Anregungslicht 124 kann ebenso wie das Fluoreszenzlicht 125 über Faseroptiken 122 und verschiedene optische Elemente wie Strahlteiler, Linsen oder Spiegel 126, 128 geleitet werden. Das Fluoreszenzlicht wird schließlich von einem Photodetektor 130 detektiert und das Signal in weiteren Signalverarbeitungsstufen 132 bis 136 nachverarbeitet, wie etwa einem Differenzverstärker, einem AD-Wandler, Frequenzfiltern und Mustererkennungseinheiten.

Zusätzlich oder alternativ zu einer Gradiometerkonfiguration kann für ein Magnetometer eine Lock-In-Detektion genutzt werden, um die unerwünschten Hintergrundfelder aus der Messung zu eliminieren.

Das Sensormedium, d.h. der Sensordiamant, kann beispielsweise ein dünnes Diamantplättchen umfassen, da kein großes Volumen zur Fluoreszenzmessung notwendig ist. Damit kann ein Sensor noch kompakter und auch kostengünstiger gestaltet werden. Alternativ oder zusätzlich kann das Sensormedium der beiden Magnetometer auch jeweils einen Abschnitt desselben Diamantkristalls umfassen. In dieser Bauweise ist, neben der Kompaktheit, auch sichergestellt, dass die beiden als Sensormedium genutzten Abschnitte im Wesentlichen gleiche Eigenschaften aufweisen und damit auch die gleichen optischen Eigenschaften bei der Auslesung der Spinresonanz zeigen, so dass keine Fehlerquellen bei der Differenzbildung des Gradiometers entstehen.

Als weitere Ausführungsform können auch Gradiometereinheiten zur Fahrerzustandsüberwachung aus zwei Dampfzellen-Magnetometern gebildet werden. Während diese Dampfzellen-Magnetometer oft eine starke magnetische Abschirmung benötigen, da sie üblicherweise nur bei kleinen Feldstärken nahe dem feldfreien Bereich (near-zero field) betrieben werden, können mit einer Gradiometerkonfiguration oder mit geeigneter Lock-In-Detektion auch Messungen in Normalumgebung mittels Dampfzellen vorgenommen werden. Ebenso sind auch Kombinationen aus Dampfzellen und NV-Zentren-Magnetometern innerhalb einer Gradiometereinheit möglich.

Um durch Hirnströme induzierte Magnetfelder auszulesen, können dann mehrere einzelne Magnetometer und/oder mehrere Gradiometer-Einheiten aus jeweils zwei Magnetometern an verschiedenen Stellen rund um den Kopf eines Fahrers in einem Helm angeordnet werden, um zusammen eine Sensoreinheit zu bilden. Entsprechend ihrer spezifischen räumlichen Anordnung addieren sich die von einzelnen Neuronen erzeugten Potentiale und die induzierten Magnetfelder auf, so dass sich über den gesamten Kopf verteilte Magnetfeldänderungen messen lassen.

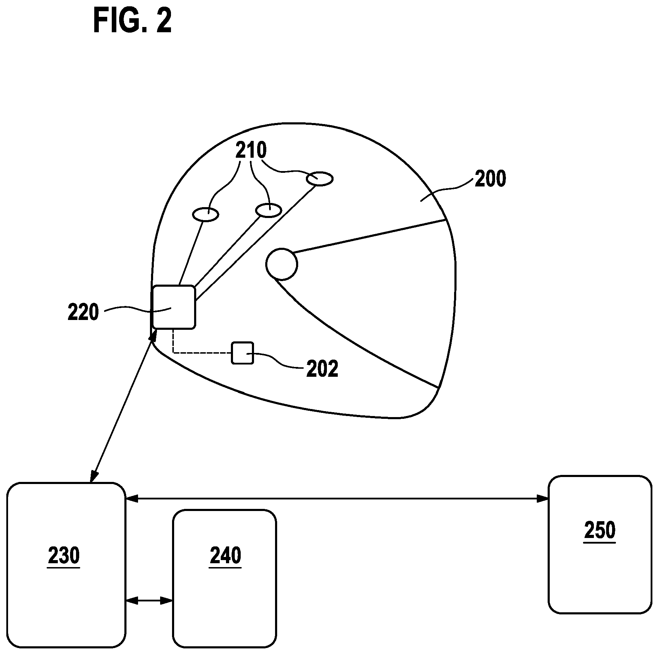

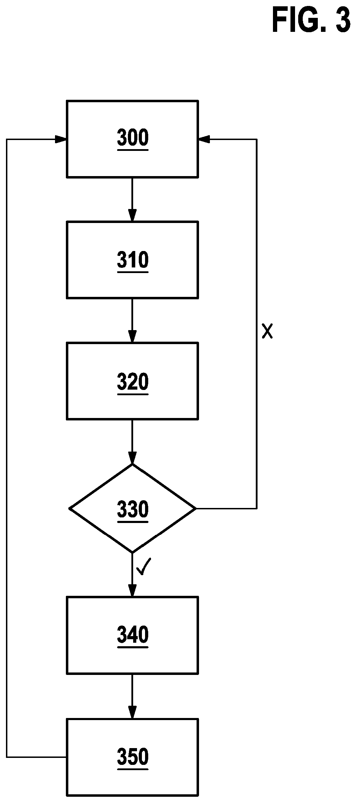

Ein beispielhaftes Sensorsystem mit einem Motorradhelm 200 ist schematisch in Figur 2 gezeigt und wird in Bezug auf die Verfahrensschritte in Figur 3 beschrieben. Mehrere Sensoreinheiten 210, wie etwa die oben beschriebenen Magnetfeld-Gradiometer, sind an verschiedenen Stellen um den Kopf eines Benutzers im Helm integriert. Dabei sind die Sensoreinheiten im Helm nur beispielhaft gezeigt, so dass die exakten Orte, an denen die Sensoreinheiten relativ zum Kopf innerhalb des Helms angebracht sind, auch völlig anders gewählt werden können, bevorzugt abhängig von den Hirnregionen, in denen zustandsabhängige neuronale Aktivitäten erwartet werden. Die für den Betrieb der Sensoreinheiten nötigen Bauteile werden hier nicht detailliert aufgeführt; es versteht sich aber, dass je nach Art der Sensoren hier beispielsweise eine Stromversorgung wie ein Akku, eine Magnetfeldquelle, eine Anregungslichtquelle, Photodetektoren und andere Elemente kompakt im Helm integriert sein können. Mit diesen Sensoreinheiten im Helm können Hirn-Magnetfeldsignale eines Benutzers in Schritt 300 des in Figur 3 gezeigten Verfahrens aufgezeichnet werden.

Da kein direkter Kontakt zur Kopfhaut notwendig ist, kann die innere Helmoberfläche so gestaltet sein, dass die Sensoreinheiten 210 verdeckt liegen, z.B. durch eine Innenfläche aus einem Kunststoff oder einem stabilen Textilmaterial, die am Kopf des helmtragenden Benutzers anliegt. Dazu können die Sensoreinheiten beispielsweise in die Helmpolster oder entsprechende Halterungen im Helmmaterial integriert sein. Dabei ist bevorzugt, dass die Sensorköpfe der Sensoreinheiten so nah wie möglich an der Innenfläche zu liegen kommen, um den Abstand zum Kopf und damit zu den Hirnbereichen als Magnetfeldquellen möglichst gering zu halten. Gleichzeitig kann eine stabile überdeckende Helminnenfläche einen mechanischen Schutz für die empfindlichen Sensorelemente bieten.

Die Sensoreinheiten 210 sind mit einer Helm-Verarbeitungseinheit 220 verbunden, an die die erfassten Sensordaten übermittelt werden. Diese Einheit kann beliebig einfach oder komplex gestaltet sein. Beispielsweise könnte es sich um ein Kommunikationsmodul handeln, das alle von den Sensoreinheiten erfassten Sensordaten im Wesentlichen unverändert an eine externe Verarbeitungseinheit 230 weiterleitet, in der dann weitere Verarbeitungs- und Auswertungsschritte vorgenommen werden können. Dazu eignet sich beispielsweise bevorzugt eine drahtlose, kurzreichweitige Kommunikationsschnittstelle, wie etwa Bluetooth oder WLAN. Grundsätzlich ist aber auch eine Datenübertragung über größere Entfernungen, z.B. an eine Zentraleinheit des Herstellers oder Anbieters denkbar, soweit entsprechende Kommunikationsverbindungen über eine Mobilfunkverbindung oder andere Mittel möglich sind.

Alternativ können in der Helm-Verarbeitungseinheit auch weitere Schritte vorgenommen werden. Beispielsweise kann dort in Schritt 310 eine elektronische Signal-Vorverarbeitung durch Filter, Verstärker, oder andere Elemente stattfinden. Falls eine ausreichend kompakte und leistungsfähige Auswertungseinheit in Form z.B. eines Prozessors mit Speicherelementen und geeigneter Software vorliegt, die in den Helm integriert werden kann, kann auch diese als Teil der Helm-Verarbeitungseinheit 220 vorgesehen sein bzw. damit verbunden sein. Eine solche Auswertungseinheit kann dann je nach Ausgestaltung weitere Auswertungsschritte übernehmen, die im nachfolgenden noch genauer beschrieben werden.

In vielen Fällen bietet sich aber an, die eigentliche Auswertung 320, 330 der Sensordaten außerhalb des Helms in der externen Verarbeitungseinheit 230 vorzunehmen. Diese externe Verarbeitungseinheit 230 kann ein Hardware- und/oder Softwaremodul umfassen, das spezifisch nur für die Zustandsüberwachung und die Verarbeitung der Sensordaten genutzt wird. Alternativ können als externe Verarbeitungseinheit aber auch bereits vorhandene Einheiten verwendet werden, indem diese mit entsprechenden Softwaremodulen und optional mit weiterer Hardware ergänzt werden. Beispielsweise kann die Auswertung durch eine Anwendung vorgenommen werden, die in einem tragbaren elektronischen Gerät wie einem Smartphone oder einem Tabletcomputer installiert und ausgeführt wird.

Die Auswertungseinheit, unabhängig davon, ob sie im Helm oder extern untergebracht ist, dient zur Klassifizierung der Sensordaten in Bezug auf bekannte oder unbekannte Benutzerzustände. Zur Auswertung der erfassten Hirn-MagnetfeldSignale können klassische Methoden wie verschiedene Mustererkennungsverfahren, einfache Grenzwerte, zeitliche Verläufe, Toleranzbereiche, Steigungen, Frequenzauswertungen und andere zur Anwendung kommen. Die jeweiligen Referenzwerte können im System abgespeichert sein und bestimmten Zuständen oder Erkrankungen zugeordnet sein, wie etwa Müdigkeit, Stress, Schlaf, aber auch neuronalen Erkrankungen wie Parkinson, Epilepsie oder z.B. Schlaganfällen.

Alternativ oder zusätzlich kann an dieser Stelle als Auswertungseinheit auch künstliches/maschinelles Lernen z.B. in Form eines neuronalen Netzes eingesetzt werden, um die gemessenen Sensordaten auszuwerten und geeignet zu klassifizieren. Beispielsweise kann ein neuronales Netz ebenfalls mit im Labor gewonnenen Daten trainiert sein, die optional auch durch weitere Messmethoden wie EEG als Referenz bewertet wurden. Anhand dieser Daten kann dann eine künstlich lernende Einheit wiederum beispielsweise Müdigkeit, Stress oder Erkrankungen erkennen. Zur Gewinnung der Referenz- bzw. Trainingsdaten kann ebenfalls ein Sensorsystem in einem Helm verwendet werden, um möglichst vergleichbare Daten zu erzeugen.

Die in Schritt 300 erfassten und gegebenenfalls in Schritt 310 vorverarbeiteten und übermittelten Sensordaten können also in Schritt 320 aus Figur 3 in einer Auswertungseinheit klassifiziert werden. Die Sensordaten werden dabei als Eingabewerte an die Auswertungseinheit gegeben. Anhand der Ausgabewerte, also dem Ergebnis der Klassifizierung, die einen erkannten Zustand eines Benutzers angeben, kann in Schritt 330 geprüft werden, ob es sich um einen unbekannten oder unerwünschten Zustand handelt.

Da es zwischen unterschiedlichen Benutzern eine gewisse Streuung in den repräsentativen Hirnstrommustern gibt, können insbesondere bei Nutzung einer künstlich lernenden Auswertungseinheit auch aktuelle Sensordaten eines Benutzers gespeichert werden und mit in die weitere Auswertung einbezogen werden. Zu diesem Zweck können Daten aus dem laufenden Anwendungsbetrieb einer Sensoreinheit beispielsweise als nutzerspezifische Trainingsdaten für ein neuronales Netz eingesetzt werden. So erhält man eine plastische Auswertungseinheit, deren Parameter wie etwa die Verbindungsgewichte noch nachträglich im Betrieb verändert werden können. Es können auch mehrere künstlich lernende Auswertungseinheiten kombiniert werden, so dass beispielsweise ein statisches, unverändertes neuronales Netz und ein plastisches, kontinuierlich nachtrainiertes neuronales Netz verwendet werden. Auch bei einer klassischen Musterauswertung können grundsätzlich noch aktuelle Daten mit einfließen und z.B. durch Änderung bestimmter vorgegebener Grenzwerte oder Parameter für die zukünftige Auswertung der gemessenen Magnetfeldmuster genutzt werden.

Abhängig von den in der Auswertung 320 erkannten Zuständen des Fahrers können dann geeignete Reaktionen in Schritt 340 gewählt und in Schritt 350 ausgelöst werden. Beispielsweise können bei erkannter Müdigkeit optische oder akustische Signale ausgegeben werden, die zur Aufmerksamkeit beitragen oder dem Fahrer z.B. in Form einer Text- oder Sprachausgabe vorschlagen, eine Pause einzulegen. Solche Signale können beispielsweise über Lautsprecher ausgegeben werden, die im Helm angebracht sind. Daneben könnten auch Signale am Fahrzeug aktiviert werden, z.B. durch Aktivieren einer Warnblinkanlage, um andere Fahrzeuge auf einen Notfall aufmerksam zu machen. Falls die Signale nicht direkt von der Auswertungseinheit 230 ausgegeben werden, können diese bzw. ein entsprechender Befehl an eine andere Einheit 240, z.B. ein Steuergerät des Fahrzeugs, übermittelt werden.

Bei kritischen gesundheitlichen Zuständen könnte dagegen eine Kommunikationsverbindung mit entfernten Einheiten 250 aufgenommen werden, so dass zum Beispiel ein Notruf an eine Zentrale, an eine vorgegebene Kontaktperson oder an eine Rettungsleitstelle übermittelt werden. Auch eine direkte uni- oder bidirektionale Sprechverbindung zum Fahrer zum Abklären des tatsächlichen Zustands ist möglich, soweit im Helm 200 geeignete Schnittstellen wie Mikrofon und Lautsprecher vorhanden sind. Ebenso könnte eine Kommunikationsmöglichkeit zu anderen Fahrzeugen genutzt werden (Vehicle-to-Vehicle), um diese auf den eingetretenen Notfall oder auf unerwartete Fahrmanöver hinzuweisen. Außerdem können die erfassten Sensordaten und/oder die daraus abgeleiteten Benutzerzustände auch zur Speicherung oder zur weiteren Auswertung an andere Einheiten übermittelt werden. Diese und weitere geeignete Reaktionen können auch beliebig miteinander kombiniert werden.

Mit den bereits beschriebenen Auswertungen durch künstlich lernende Einheiten sind auch Ausführungen möglich, die in der Lage sind, zu den Klassifizierungen der Sensordaten auch ein Maß für eine Unsicherheit dieser Ausgabewerte anzugeben. In diesem Fall können die Reaktionen nicht nur abhängig von dem erkannten Zustand, sondern auch abhängig von dem Maß der Unsicherheit gemacht werden, z.B. durch Festlegung von bestimmten Schwellwerten, die erfüllt sein müssen, um Fehlalarme zu verhindern. Außerdem kann ein Maß für die Unsicherheit der Zustandsklassifizierung zur Festlegung von Gewichtungsfaktoren genutzt werden, wenn mehrere Auswertungseinheiten vorhanden sind. Die Gesamtausgabe, die die Reaktion bestimmt, kann dann aus einer gewichteten Summe der einzelnen Ergebnisse gebildet werden.

Es kann außerdem eine Schaltfunktion vorgesehen sein, welche die Sensoreinheiten 210 und die Helm-Verarbeitungseinheit 220 betriebsfähig einschaltet, also z.B. für ein NV-Zentren-Gradiometer alle erforderlichen Elemente wie Anregungslichtquelle, Mikrowellenquelle oder Signalverarbeitungselektronik aktiviert. Die Schaltfunktion kann beispielsweise durch einen manuellen Schalter 202 am Helm 200 vorgesehen sein. Alternativ oder zusätzlich kann auch eine drahtlose Verbindung (z.B. eine energiesparende kurzreichweitige Funkverbindung wie Bluetooth LE) zu einem Steuergerät eines Motorrads vorgesehen sein, über die die Sensoreinheiten im Helm aktiviert werden, sobald das Fahrzeug gestartet wird. Denkbar ist auch ein zusätzlicher Sensor, der erkennt, wenn der Helm bewegt oder aufgesetzt wird und in Reaktion darauf die Sensoreinheiten und Helm-Verarbeitungseinheiten aktiviert.

Durch die während einer Fahrt wirkenden Kräfte kann sich der Helm und damit auch die Position der Sensoreinheiten im Helm gegenüber dem Kopf, also gegenüber den durch Hirnaktivität erzeugten Magnetfeldquellen, leicht verschieben.

Um diese Verschiebung bei der Auswertung der Magnetfeldmessungen zu berücksichtigen, können daher verschiedene weitere Parameter und Daten mit einbezogen werden. Beispielsweise können Geschwindigkeits- und Beschleunigungsdaten während der Fahrt erfasst werden. Auf Basis dieser Werte und bekannter Parameter des Helms (z.B. Gewicht) können dann die wirkenden Kräfte und die möglichen Verschiebungen grob modelliert werden. Zu diesem Zweck können insbesondere Daten verwendet werden, die in einem Steuergerät des Fahrzeugs bereits direkt oder indirekt vorhanden sind, z.B. die aktuelle Fahrtgeschwindigkeit. Die Daten können beispielsweise von dem Steuergerät 240 durch die Auswertungseinheit 230 regelmäßig abgefragt werden.

Optional können solche Messdaten auch durch weitere Informationen ergänzt werden, die indirekte Hinweise auf den Fahrtverlauf und die Beschleunigungen bieten. Beispielsweise könnte anhand von Straßenkarten mit Kurven und Abbiegungen der erwartete Fahrtverlauf modelliert oder berechnet werden. Ebenso ist denkbar, weitere Sensordaten von anderen Sensoren zu erfassen, die eine Vorhersage über die Positionsverschiebung der Sensoreinheiten am Kopf ermöglichen, wie z.B. Inertialsensoren, ABS-, ESP-Sensoren usw.

Eine weitere Möglichkeit zur Korrektur des Signals bei Verschiebungen der Sensoreinheiten gegenüber dem Kopf besteht in der Beobachtung eines vorgegebenen weiteren Hirnsignals, welches nicht durch den Benutzerzustand (z.B. Müdigkeit) beeinflusst wird, also bevorzugt ein Signal, das sich nur durch die Kopfposition gegenüber dem Sensor verändert. Ein solches Signal kann dann als Referenzsignal für die Position des Kopfes gegenüber der Sensoreinheit verwendet werden. Signale unterschiedlicher Frequenzen können dabei wiederum durch geeignete Signalverarbeitung, wie etwa Filter oder eine Lock-In-Detektion, unterschieden werden.

Die Magnetfeld-Sensordaten aus dem Helm können auch durch weitere erfasste Daten aus separaten Sensoren und Modulen ergänzt werden, wie etwa Kamerabilder einer Kamera, die im Augenbereich des Helms störungsfrei angebracht ist, um daraus Blickrichtungen, Augenbewegungen oder Lidaufschläge auszuwerten. Auch eigenständige Vorrichtungen wie etwa am Arm getragene Einheiten zur Pulsüberwachung können Sensordaten ausgeben, die weitere Hinweise zum Zustand eines Benutzers liefern.

Zusätzlich kann der Helm auch beispielsweise mit Gyroskopen und/oder Beschleunigungssensoren ausgestattet sein, die Hinweise auf einen Sturz des Benutzers geben können. Außerdem können mit solchen Sensoren im Helm Kopfbewegungen des Benutzers ausgewertet werden, die auf bestimmte Zustände hinweisen. Dabei können durch kontinuierliches Training einer Auswertungseinheit auch typische Kopfbewegungen eines Benutzers mit einbezogen werden. Die Gyroskope können auf ähnliche Weise wie die Magnetfeldsensoren im Helm integriert sein, wobei hier eine Anordnung an der Helminnenfläche keine Rolle spielt.

Dabei kommen grundsätzlich alle Typen von Gyroskopen in Betracht. Besonders geeignet sind beispielsweise die weit verbreiteten MEMS-Drehratensensoren (microelectromechanical system), die klein und ausreichend empfindlich sind. Diese Sensoren sind üblicherweise als komplettes Sensorsystem ähnlich einer integrierten Schaltung gebaut und können Sensoren für eine oder mehrere Achsen umfassen. Ebenso können aber auch andere Gyroskope zur Zustandsüberwachung verwendet werden. Auf ähnliche Weise wie für die Anwendung als Magnetometer beschrieben, können Dampfzellen und NV-Zentren in Diamant auch zur spinbasierten optischen Messung von Drehraten genutzt werden und hochempfindliche Gyroskope bilden. Beispielsweise offenbart die DE 10 2019 219 061 A1 eine Möglichkeit zur Messung von drei Rotationsrichtungen mittels eines NMR-Gyroskops.

Die durch das Helm-Sensorsystem erfassten Daten und erkannten Zustände können mit weiteren Systemen kombiniert werden, wie etwa einem Sturzsensor im Motorrad oder einem eCall-System zum Auslösen eines Notrufs bei erkannten Kollisionen und Stürzen.

Es versteht sich, dass die vorstehend beschriebenen und gezeigten Ausführungsbeispiele nur zur Verdeutlichung des Erfindungsprinzips dienen sollen und die Erfindung nicht auf diese Varianten beschränkt ist. Beispielsweise können verschiedene der Funktionen, für die getrennte Funktionseinheiten beschrieben wurden, auch teilweise von einer einzigen Einheit übernommen werden oder anders in mehrere Untereinheiten kombiniert werden. Entsprechend können auch verschiedene Einheiten anders angeordnet sein, so dass beispielsweise ein Teil der Signalauswertung und Klassifizierung im Helm selbst in einer kompakten Auswertungseinheit mit geeignetem Prozessor und Speichermodul stattfinden kann, während ein weiterer Teil in der externen Auswertungseinheit vorgenommen wird. Alternativ könnten alle Daten an ein Modul übertragen werden, das in die Fahrzeugsteuerung integriert ist, so dass also die externe Auswertungseinheit 230 und das Steuergerät 240 des Fahrzeugs als eine Einheit ausgeführt sind.

Außerdem versteht sich, dass die hier beschriebenen Beispiele nicht auf Motorradhelme beschränkt sind, sondern problemlos auf andere Bereiche übertragen werden kann, in denen ebenfalls Helme getragen werden. Beispielsweise bietet sich eine entsprechende Umsetzung auch in einem Pilotenhelm für Piloten eines Flugzeugs oder Hubschraubers oder in einem Helm für Rennfahrer an. Alle Elemente, die hier für Motorräder beschrieben sind, können dann ebenso in das jeweilige benutzte Fahrzeug integriert werden.

## Patentansprüche

1. Verfahren zur Zustandsüberwachung, umfassend:
Erfassen von Sensordaten im Kopfbereich eines Benutzers während einer Fahrzeugfahrt, die abhängig von einem mentalen oder gesundheitlichen Zustand des Benutzers sind, wobei die Sensordaten über eine oder mehrere Sensoreinheiten (210) erfasst werden, die in einem Helm (200), der von dem Benutzer getragen wird, angeordnet sind;
Prüfen, ob die erfassten Sensordaten auf einen unerwünschten oder unbekannten Zustand des Benutzers hinweisen, der die Fahrtüchtigkeit des Benutzers einschränkt;
und falls dies der Fall ist, Auslösen einer zustandsabhängigen Reaktion;
weiter umfassend:
Erfassen von Fahrtdaten der Fahrzeugfahrt;
Ermitteln einer erwarteten Auswirkung der Fahrtdaten auf eine Position des Helms (200) relativ zum Kopf des Benutzers, und
Bestimmen einer Positionskorrektur für die erfassten Sensordaten auf Basis der Fahrtdaten.

2. Verfahren nach Anspruch 1, wobei die zustandsabhängige Reaktion mindestens eines der folgenden umfasst: ein Ausgeben eines optischen und/oder akustischen Signals an den Benutzer, ein Übermitteln einer Nachricht über eine Kommunikationsschnittstelle an eine Notrufzentrale, ein Übermitteln einer Nachricht über eine Kommunikationsschnittstelle an andere Fahrzeuge in der Umgebung.

3. Verfahren nach Anspruch 2, wobei ein optisches und/oder akustisches Signal durch Signalisierungsmittel ausgegeben wird, die in dem Helm (200) angeordnet sind.

4. Verfahren nach einem der vorhergehenden Ansprüche, wobei das Prüfen der erfassten Sensordaten in einer externen Verarbeitungseinheit (230) außerhalb des Helms (200) als Auswertungseinheit erfolgt, und wobei das Verfahren weiter ein Übermitteln der erfassten Sensordaten an die Auswertungseinheit über ein Kommunikationsmodul (220) in dem Helm (200) umfasst.

5. Verfahren nach einem der vorhergehenden Ansprüche, wobei die Fahrtdaten mindestens eines der folgenden umfassen: eine aktuelle Fahrgeschwindigkeit, eine aktuelle Beschleunigung, ein vorgegebener Fahrweg auf einer Karte.

6. Verfahren nach einem der vorhergehenden Ansprüche, wobei der unerwünschte Zustand des Benutzers mindestens eines der folgenden umfasst: Müdigkeit, Einschlafen, erhöhtes Stressniveau, niedrige Konzentration, eine neuronale Erkrankung, einen epileptischen Anfall, einen Schlaganfall.

7. Sensorsystem zum Überwachen eines Zustands eines Benutzers, aufweisend:
einen Helm (200), der zum Tragen durch einen Benutzer während einer Fahrzeugfahrt ausgelegt ist,
wobei in dem Helm (200) eine oder mehrere Sensoreinheiten (210) angeordnet sind, welche dazu eingerichtet sind, Sensordaten im Kopfbereich des Benutzers zu erfassen, die abhängig von einem mentalen oder gesundheitlichen Zustand des Benutzers sind;
wobei das System weiter eine Auswertungseinheit aufweist, die dazu eingerichtet ist zu prüfen, ob die erfassten Sensordaten auf einen unerwünschten oder unbekannten Zustand des Benutzers hinweisen, der die Fahrtüchtigkeit des Benutzers einschränkt, und falls dies der Fall ist, eine zustandsabhängige Reaktion auszulösen
weiter aufweisend eine Korrektureinheit, die dazu eingerichtet ist, Fahrtdaten der Fahrzeugfahrt zu erfassen, eine erwartete Auswirkung der Fahrtdaten auf eine Position des Helms (200) am Kopf eines Benutzers zu ermitteln und auf Basis der Fahrtdaten eine Positionskorrektur für die erfassten Sensordaten zu bestimmen.

8. Sensorsystem nach Anspruch 7, wobei die Auswertungseinheit in einer externen Verarbeitungseinheit (230) außerhalb des Helms (200) vorliegt, und wobei der Helm (200) weiter ein Kommunikationsmodul aufweist, der zum Übermitteln der Sensordaten an die Auswertungseinheit eingerichtet ist.

9. Sensorsystem nach Anspruch 7 oder 8, weiter aufweisend eine Kommunikationsschnittstelle zum Übermitteln einer Nachricht durch die Auswertungseinheit an eine Notrufzentrale (250) oder an mindestens ein weiteres Kommunikationsmodul eines weiteren Fahrzeugs.

10. Sensorsystem nach einem der Ansprüche 7 bis 9, weiter aufweisend eine Kommunikationsschnittstelle, die zum Datenaustausch mit einem Steuergerät (240) eines Fahrzeugs eingerichtet ist.

11. Sensorsystem nach einem der Ansprüche 7 bis 10, wobei die eine oder eine der mehreren Sensoreinheiten (201) ein spinresonanz-basiertes Magnetometer zum Erfassen von durch Hirnströmen induzierten Hirn-Magnetfeldern aufweist.

12. Sensorsystem nach einem der Ansprüche 7 bis 11, wobei die eine oder eine der mehreren Sensoreinheiten (201) zwei Magnetometer aufweist, die in einem festen Abstand zueinander angeordnet sind,
wobei jedes Magnetometer ein Sensormedium aufweist und dazu eingerichtet ist, eine magnetische Feldstärke an einem Messort im Kopfbereich des Benutzers durch Auslesen einer von der magnetischen Feldstärke abhängigen Spinresonanz in dem Sensormedium zu erfassen,
wobei die Sensoreinheit weiter
mindestens eine Anregungslichtquelle zum Einstrahlen von Licht in die Sensormedien (101, 102) der Magnetometer aufweist,
und mindestens eine Signalverarbeitungseinheit zum Ermitteln eines Magnetfeldgradienten an einer Sensoreinheit (100, 200, 300, 400) als Differenz der Ausgangssignale der zwei Magnetometer der Sensoreinheit und zum Ausgeben der Differenz als Sensordaten der Sensoreinheit.

13. Recheneinheit, die dazu eingerichtet ist, alle Verfahrensschritte eines Verfahrens nach einem der Ansprüche 1 bis 5 durchzuführen.

14. Computerprogramm, das eine Recheneinheit dazu veranlasst, alle Verfahrensschritte eines Verfahrens nach einem der Ansprüche 1 bis 5 durchzuführen, wenn es auf der Recheneinheit ausgeführt wird.

15. Maschinenlesbares Speichermedium mit einem darauf gespeicherten Computerprogramm nach Anspruch 14.

## Claims

1. Method for condition monitoring, comprising:
collecting sensor data in the head region of a user during a vehicle journey, which sensor data are dependent on a mental or physical condition of the user, the sensor data being collected by means of one or more sensor units (210) arranged in a helmet (200) worn by the user;
checking whether the collected sensor data indicate an undesirable or unknown condition of the user that limits the user's fitness to drive;
and, if this is the case, triggering a condition-dependent reaction;
also comprising:
collecting journey data relating to the vehicle journey;
determining an expected effect of the journey data on a position of the helmet (200) in relation to the user's head, and
ascertaining a position correction for the collected sensor data on the basis of the journey data.

2. Method according to Claim 1, wherein the condition-dependent reaction comprises at least one of the following: output of a visual and/or audible signal to the user, transmission of a message to an emergency call centre via a communication interface, transmission of a message to other vehicles in the surroundings via a communication interface.

3. Method according to Claim 2, wherein a visual and/or audible signal is output by signalling means arranged in the helmet (200).

4. Method according to one of the preceding claims, wherein the collected sensor data are checked in an external processing unit (230) outside the helmet (200) as an evaluation unit, and wherein the method also comprises transmitting the collected sensor data to the evaluation unit via a communication module (220) in the helmet (200).

5. Method according to one of the preceding claims, wherein the journey data comprise at least one of the following: a current speed of travel, a current acceleration, a predetermined route on a map.

6. Method according to one of the preceding claims, wherein the undesirable condition of the user comprises at least one of the following: fatigue, falling asleep, increased stress level, low concentration, a neurological disorder, an epileptic seizure, a stroke.

7. Sensor system for monitoring a condition of a user, comprising:
a helmet (200) designed to be worn by a user during a vehicle journey;
the helmet (200) containing one or more sensor units (210) configured to collect sensor data in the head region of the user, which sensor data are dependent on a mental or physical condition of the user;
the system also comprising an evaluation unit configured to check whether the collected sensor data indicate an undesirable or unknown condition of the user that limits the user's fitness to drive, and, if this is the case, to trigger a condition-dependent reaction,
also comprising a correction unit configured to collect journey data relating to the vehicle journey, to determine an expected effect of the journey data on a position of the helmet (200) on a user's head and to take the journey data as a basis for ascertaining a position correction for the collected sensor data.

8. Sensor system according to Claim 7, the evaluation unit being present in an external processing unit (230) outside the helmet (200), and the helmet (200) also comprising a communication module configured for transmitting the sensor data to the evaluation unit.

9. Sensor system according to Claim 7 or 8, also comprising a communication interface for transmitting a message by the evaluation unit to an emergency call centre (250) or to at least one further communication module of a further vehicle.

10. Sensor system according to one of Claims 7 to 9, also comprising a communication interface configured for data interchange with a control unit (240) of a vehicle.

11. Sensor system according to one of Claims 7 to 10, the one, or one, of the multiple sensor units (201) comprising a spin-resonance-based magnetometer for detecting magnetic fields of the brain induced by brain currents.

12. Sensor system according to one of Claims 7 to 11, the one, or one, of the multiple sensor units (201) comprising two magnetometers arranged at a fixed distance from one another,
each magnetometer having a sensor medium and being configured to detect a magnetic field strength at a measurement location in the head region of the user by reading a magnetic-field-strength-dependent spin resonance in the sensor medium,
the sensor unit also comprising
at least one excitation light source for introducing light into the sensor media (101, 102) of the magnetometers,
and at least one signal processing unit for determining a magnetic field gradient on a sensor unit (100, 200, 300, 400) as the difference between the output signals of the two magnetometers of the sensor unit and for outputting the difference as sensor data of the sensor unit.

13. Computing unit configured to perform all the method steps of a method according to one of Claims 1 to 5.

14. Computer program that prompts a computing unit to perform all the method steps of a method according to one of Claims 1 to 5 when it is executed on the computing unit.

15. Machine-readable storage medium with a computer program according to Claim 14 stored thereon.

## Revendications

1. Procédé de surveillance d'état, comprenant :
l'acquisition de données de capteur dans la région de la tête d'un utilisateur pendant un déplacement de véhicule, lesquelles dépendent d'un état mental ou de santé de l'utilisateur, les données de capteur étant acquises par l'intermédiaire d'une ou plusieurs unités de capteur (210) qui sont agencées dans un casque (200) porté par l'utilisateur ;
la vérification du fait de savoir si les données de capteur acquises indiquent un état indésirable ou inconnu de l'utilisateur, lequel état limite l'aptitude à la conduite de l'utilisateur ;
et, si tel est le cas, le déclenchement d'une réaction dépendant de l'état ;
comprenant en outre :
l'acquisition de données de déplacement relatives au déplacement de véhicule ;
la détermination d'un effet attendu des données de déplacement sur une position du casque (200) par rapport à la tête de l'utilisateur, et
la détermination d'une correction de position pour les données de capteur acquises sur la base des données de déplacement.

2. Procédé selon la revendication 1, dans lequel la réaction dépendant de l'état comprend au moins l'une des opérations suivantes : l'émission d'un signal optique et/ou acoustique vers l'utilisateur, la transmission d'un message par l'intermédiaire d'une interface de communication à un centre d'appels d'urgence, la transmission d'un message par l'intermédiaire d'une interface de communication à d'autres véhicules dans l'environnement.

3. Procédé selon la revendication 2, dans lequel un signal optique et/ou acoustique est émis par des moyens de signalisation agencés dans le casque (200).

4. Procédé selon l'une des revendications précédentes, dans lequel la vérification des données de capteur acquises s'effectue dans une unité de traitement externe (230) à l'extérieur du casque (200) en tant qu'unité d'évaluation, et le procédé comprenant en outre la transmission des données de capteur acquises à l'unité d'évaluation par l'intermédiaire d'un module de communication (220) dans le casque (200).

5. Procédé selon l'une des revendications précédentes, dans lequel les données de déplacement comprennent au moins l'un des éléments suivants : une vitesse de déplacement actuelle, une accélération actuelle, un trajet de déplacement prédéfini sur une carte.

6. Procédé selon l'une des revendications précédentes, dans lequel l'état indésirable de l'utilisateur comprend au moins l'un des éléments suivants : de la fatigue, un endormissement, une augmentation du niveau de stress, une faible concentration, une maladie neuronale, une crise d'épilepsie, un accident vasculaire cérébral.

7. Système de capteur pour surveiller l'état d'un utilisateur, comportant :
un casque (200) conçu pour être porté par un utilisateur pendant un déplacement de véhicule,
une ou plusieurs unités de capteur (210) étant agencées dans le casque (200), lesquelles sont conçues pour acquérir, dans la région de la tête de l'utilisateur, des données de capteur qui dépendent d'un état mental ou de santé de l'utilisateur ;
le système comprenant en outre une unité d'évaluation qui est conçue pour vérifier si les données de capteur acquises indiquent un état indésirable ou inconnu de l'utilisateur, lequel limite l'aptitude à la conduite de l'utilisateur et, si tel est le cas, pour déclencher une réaction dépendant de l'état,
comportant en outre une unité de correction qui est conçue pour acquérir des données de déplacement de véhicule, pour déterminer un effet attendu des données de déplacement sur une position du casque (200) au niveau de la tête d'un utilisateur et pour déterminer une correction de position pour les données de capteur acquises sur la base des données de déplacement.

8. Système de capteur selon la revendication 7, dans lequel l'unité d'évaluation est présente dans une unité de traitement externe (230) à l'extérieur du casque (200), et dans lequel le casque (200) présente en outre un module de communication qui est conçu pour transmettre les données de capteur à l'unité d'évaluation.

9. Système de capteur selon la revendication 7 ou 8, comportant en outre une interface de communication permettant la transmission d'un message par l'unité d'évaluation à un centre d'appels d'urgence (250) ou à au moins un autre module de communication d'un autre véhicule.

10. Système de capteur selon l'une des revendications 7 à 9, comportant en outre une interface de communication qui est conçue pour échanger des données avec un dispositif de commande (240) d'un véhicule.

11. Système de capteur selon l'une des revendications 7 à 10, dans lequel ladite une ou l'une de la pluralité d'unités de capteur (201) comporte un magnétomètre à base de résonance de spin permettant d'acquérir des champs magnétiques cérébraux induits par des courants cérébraux.

12. Système de capteur selon l'une des revendications 7 à 11, dans lequel ladite une ou l'une de la pluralité d'unités de capteur (201) comporte deux magnétomètres qui sont agencés à une distance fixe l'un de l'autre,
chaque magnétomètre comportant un milieu capteur et étant conçu pour acquérir une intensité de champ magnétique à un emplacement de mesure dans la région de la tête de l'utilisateur par lecture, dans le milieu capteur, d'une résonance de spin qui dépend de l'intensité de champ magnétique, l'unité de capteur comportant en outre
au moins une source de lumière d'excitation destinée à émettre de la lumière dans les milieux capteurs (101, 102) des magnétomètres,
et au moins une unité de traitement de signaux destinée à déterminer un gradient de champ magnétique au niveau d'une unité de capteur (100, 200, 300, 400) sous la forme d'une différence entre les signaux de sortie des deux magnétomètres de l'unité de capteur et à fournir la différence en tant que données de capteur de l'unité de capteur.

13. Unité de calcul qui est conçue pour mettre en œuvre toutes les étapes de procédé d'un procédé selon l'une des revendications 1 à 5.

14. Programme informatique qui amène une unité de calcul à mettre en œuvre toutes les étapes de procédé d'un procédé selon l'une des revendications 1 à 5, lorsqu'il est exécuté sur l'unité de calcul.

15. Support d'enregistrement lisible par machine, sur lequel est enregistré un programme informatique selon la revendication 14.
